# EUROPEAN PATENT SPECIFICATION

(11) **EP 1 210 108 B1**
(45) Date of publication and mention of the grant of the patent: **25.11.2009**
(21) Application number: 00956758.7
(22) Date of filing: 04.09.2000
(51) Int. Cl.: A61K 38/22, A61K 48/00, A61P 3/00, A61P 9/00, A61P 15/00, A61P 35/00, A61P 43/00

(54) **USE OF LEPTIN TOGETHER WITH AN INHIBITOR OF VEGF AND/OR AN INHIBITOR OF ANGIOGENESIS FOR THE INHIBITION OF ENDOTHELIAL CELL PROLIFERATION**
VERWENDUNG VON LEPTIN MIT EINEM VEGF HEMMER UND/ODER ANGIOGENESE HEMMER ZUR HEMMUNG DER PROLIFERATION ENDOTHELIALER ZELLEN
UTILISATION DE LA LEPTINE EN COMBINAISON AVEC UN INHIBITEUR DE VEGF ET/OU UN INHIBITEUR DE L'ANGIOGENESE POUR INHIBER LA PROLIFERATION DES CELLULES ENDOTHELIALES

(30) Priority: 05.09.1999 IL 13173999; 10.10.1999 IL 13231299
(43) Date of publication of application: 05.06.2002
(73) Proprietor: YEDA RESEARCH AND DEVELOPMENT CO., LTD., 76100 Rehovot (IL)
(72) Inventor: RUBINSTEIN, Menachem, 54042 Givat Shmuel (IL); COHEN, Batya, 64923 Tel Aviv (IL); BARKAN, Dalit, 76448 Rehovot (IL)
(74) Representative: Grünecker, Kinkeldey, Stockmair & Schwanhäusser Anwaltssozietät
(86) International application number: PCT/IL2000/000525
(87) International publication number: WO 2001/018040

(56) References cited:
- WO-A-97/27286
- WO-A-98/48831
- WO-A-99/59614
- SIERRA-HONIGMANN M ROCIO ET AL: "Biological action of leptin as an angiogenic factor." SCIENCE (WASHINGTON D C), vol. 281, no. 5383, 11 September 1998 (1998-09-11), pages 1683-1686, XP002161601 ISSN: 0036-8075
- LAFONTAN MAX ET AL: "Leptin and angiogenesis." M-S (MEDECINE SCIENCES), vol. 15, no. 3, March 1999 (1999-03), pages 382-386, XP000982571 ISSN: 0767-0974
- COHEN B. ET AL.: "Leptin induces Angiopoietin-2 expression in adipose tissues" J. BIOL. CHEM., vol. 276, no. 11, 16 March 2001 (2001-03-16), pages 7697-7700, USA

## Description

### Field of the Invention

The present invention relates to reversible inhibition of endothelial cell proliferation and to modulation of angiogenesis in the female reproductive system. More particularly, the present invention relates to the use of leptin or leptin homologues or derivatives, optionally together with inhibitors of VEGF action or inhibitors of VEGF synthesis, in the preparation of a medicament for inhibiting angiogenesis or modulating angiogenic processes. The present invention further relates to a pharmaceutical composition comprising leptin or a leptin homologue or derivative together with an inhibitor of VEGF action or of VEGF synthesis and/or an inhibitor of angiogenesis.

### Background of the Invention

As used herein, the term "angiogenesis" means the generation of new blood vessels into a tissue or organ. Under normal physiological conditions, humans or animals undergo angiogenesis only in very specific restricted situations. For example, angiogenesis is normally observed in wound healing, fetal and embryonic development, formation of the corpus luteum, endometrium and placenta and growth of adipose tissue.

The term "endothelium" means a thin layer of flat epithelial cells that lines serous cavities, lymph vessels, and blood vessels. Both controlled and uncontrolled angiogenesis are thought to proceed in a similar manner. Endothelial cells and pericytes, surrounded by a basement membrane, form capillary blood vessels. Angiogenesis begins with the erosion of the basement membrane by enzymes released by endothelial cells and leukocytes. The endothelial cells, which line the lumen of blood vessels, then protrude through the basement membrane. Angiogenic stimulants induce the endothelial cells to migrate through the eroded basement membrane. The migrating cells form a "sprout" off the parent blood vessel, where the endothelial cells undergo mitosis and proliferate. The endothelial sprouts merge with each other to form capillary loops, creating the new blood vessel.

Persistent, unregulated angiogenesis occurs in a multiplicity of disease states, tumor metastasis and abnormal growth by endothelial cells and supports the pathological damage seen in these conditions. The diverse pathological disease states in which unregulated angiogenesis is present have been grouped together as angiogenic dependent or angiogenic associated diseases. The hypothesis that tumor growth is angiogenesis-dependent was first proposed in 1971. (Folkman J., Tumor angiogenesis: Therapeutic implications. N. Engl. J. Med. 285:1182-1186, 1971). In its simplest terms it states: "Once tumor 'take' has occurred, every increase in tumor cell population must be preceded by an increase in new capillaries converging on the tumor." Tumor 'take' is currently understood to indicate a prevascular phase of tumor growth in which a population of tumor cells occupying a few cubic millimeters volume and not exceeding a few million cells, can survive on existing host microvessels. Expansion of tumor volume beyond this phase requires the induction of new capillary blood vessels. For example, pulmonary micrometastases in the early prevascular phase in mice would be undetectable except by high power microscopy on histological sections.

Vascular morphogenesis is regulated by the hypoxia-induced vascular endothelial growth factor (VEGF) and its endothelial cell receptors Flkl and Flt 1. Two other angiogenic factors, angiopoietin-1 and 2 (Angl and Ang2), which bind to a common endothelial cell receptor (Tie2), were identified (S. Davis, et al., Cell 87, 1161-1169, 1996; P. C. Maisonpierre, et al., Science 277, 55-60, 1997). Angl is a receptor agonist (C. Suri, et al., Science 282, 468-471, 1998), constitutively expressed in many tissues, whereas Ang2 is a receptor antagonist, whose expression is limited to sites of vascular remodeling. So far, Ang2 was identified in fetal tissues, in endothelial cells, in smooth muscle cells and in female reproductive organs of adult mice and humans (P. C. Maisonpierre, et al., Science 277, 55-60, 1997; B. Witzenbichler, P. C. Maisonpierre, P. Jones, G. D. Yancopoulos, J. M. Isner, J Biol Chem 273, 18514-18521, 1998; S. J. Mandriota, M. S. Pepper, Circ Res 83, 852-859, 1998). Both VEGF and Ang2 are up-regulated in female reproductive organs upon vascular morphogenesis, whereas only Ang2 is expressed upon blood vessel regression. Ang2 probably marks these vessels for regression by an apoptotic mechanism, although induction of apoptosis by Ang2 in cultured endothelial cells has not been obtained (B. Witzenbichler, P. C. Maisonpierre, P. Jones, G. D. Yancopoulos, J. M. Isner, J Biol Chem 273, 18514-18521, 1998; J. Holash, et al., Science 284, 1994-1998, 1999; D. Hanahan, Science 277, 48-50, 1997).

A specific antibody against VEGF reduces microvessel density and causes "significant or dramatic" inhibition of growth of three human tumors, which rely on VEGF as their sole mediator of angiogenesis (in nude mice). The antibody does not inhibit growth of the tumor cells in vitro. (Kim K J, et al., Inhibition of vascular endothelial growth factor-induced angiogenesis suppresses tumor growth in vivo. Nature 362:841-844, 1993).

A specific angiogenesis inhibitor (AGM-1470) inhibits tumor growth and metastases in vivo, but is much less active in inhibiting tumor cell proliferation in vitro. It inhibits vascular endothelial cell proliferation half-maximally at **4** logs lower concentration than it inhibits tumor cell proliferation. (Ingber D, et al., Angioinhibins: Synthetic analogues of fumagillin which inhibit angiogenesis and suppress tumor growth. Nature, 48:555-557, 1990). There is also indirect clinical evidence that tumor growth is angiogenesis dependent.

Adipose tissue microcirculation is unique within the vascular system because of the capacity of this system to grow throughout most of adult life (D. L. Crandall, G. J. Hausman, J. G. Kral, Microcirculation 4, 211-232, 1997). Indeed, brown and white adipose tissues have an extensive microvasculature and express high levels of VEGF (K. P. Claffey, W. O. Wilkison, B. M. Spiegelman, J Biol Chem 267, 16317-16322, 1992; Q. X. Zhang, et al., J Surg Res 67, 147-154, 1997). Thus, it is clear that angiogenesis plays a major role in the growth and maintenance of adipose tissue. If this angiogenic activity could be repressed or eliminated, then the adipose tissue will regress.

Obesity, defined as an excess of body fat relative to lean body mass, is associated with important psychological and medical morbidities, the latter including hypertension, elevated blood lipids, and Type II or non-insulin-dependent diabetes mellitus (NIDDM). There are 6-10 million individuals with NIDDM in the U.S., including 18% of the population of 65 years of age (Hanis et al., Ira. J. Obes., 11:275-283, 1987). Approximately 45 % of males and 70% of females with NIDDM are obese, and their diabetes is substantially improved or eliminated by weight reduction (Harris, Diabetes Care, 14(3):639-648, 1991).

Y. Zhang et al (Nature, 372, 425-431, 1994) suggest that one of the molecules which plays a key role in energy balance regulation is the ob protein also termed leptin. Zhang et al also report the cloning and sequencing of both mouse and human leptin. United Kingdom patent specification No. 2292382 relates inter alia to polypeptides, ob polypeptides or allelic variants or analogs thereof and their use for modulating body weight. In particular, GB 2292382 discloses that leptins and certain analogs thereof, such as agonists, would be useful for the treatment of obesity. Indeed, it was found that the adipocyte-derived leptin regulates food intake in rodents through its action on an hypothalamic receptor. Yet, later studies have shown that serum leptin is elevated in obese individuals and that there is a direct correlation between serum leptin and the body mass index (weight in kg divided by squared height in m.). The discrepancy between leptin's effect as an inhibitor of food intake and the high levels of leptin in obese individuals led to the theory of "leptin resistance", a term suggesting that obese individuals do not respond to their high leptin levels and maintain their high body mass. Thus it is clear that leptin by itself is not efficient in reducing the adipose tissue mass (P. Prolo, M. L. Wong, J. Licinio, Int J Biochem Cell Biol 30, 1285-1290, 1998).

There exists therefore a need for a composition and method which can inhibit the unwanted growth of blood vessels, especially into tumors and adipose tissues. The composition should also be able to modulate the formation of capillaries in other angiogenic processes, such as wound healing and reproduction. The composition and method for inhibiting angiogenesis should preferably be non-toxic and produce few side effects. If angiogenic activity could be repressed or eliminated, then tumor, although present, would not grow and adipose tissue will regress. In the disease state, prevention of angiogenesis could avert the damage caused by the invasion of the new microvascular system. Therapies directed at control of the angiogenic processes could lead to the abrogation or mitigation of these diseases.

Mice lacking leptin are infertile because leptin is required for release of gonadotropin-releasing hormone (GN-RH) from the hypothalamus. GN-RH acts on the pituitary gland and is essential for the release of the gonadotropins FSH and LH. Indeed, injection of leptin to leptin-defficient mice resuced their sterility. Feamales who have low adipose tissue mass, as the case of athletes or anorexia nervosa patients are infertile due to insufficient level of the adipose tissue-produced leptin.

One of the characteristics of the estrous cycle is ovarian angiogenesis, which takes place during the maturation of the follicle in the ovary. Rupture of the follicle and formation of the corpus luteum are associated with extensive blood vessel regression. These tissues were shown to express VEGF and Ang2. Therapies directed at control of angiogenic processes in the female reproductive system could regulate fertility.

### Summary of the Invention

The present invention relates to the use of leptin, a leptin homologue or a derivative thereof, optionally together with an inhibitor of VEGF action or of VEGF synthesis and/or an inhibitor of angiogenesis, in the preparation of a medicament for inhibiting angiogenesis by inducing Ang-2 wherein daid leptin homologue has similar activity to leptin, and wherein said leptin derivative induces endothelial inhibitory activity.

Any known pharmaceutically acceptable VEGF inhibitor may be employed in accordance with the invention.

The invention also relates to pharmaceutical compositions comprising leptin, a leptin homologue or a leptin derivative together with an inhibitor of VEGF action or VEGF synthesis and/or an inhibitor of angiogenesis, wherein said leptin homologue has substantially similar activity to leptin, and wherein said leptin derivative induces endothelial inhibitory activity.

Preferably the composition is employed in angiogenesis mediated diseases.

### Brief Description of the Figures

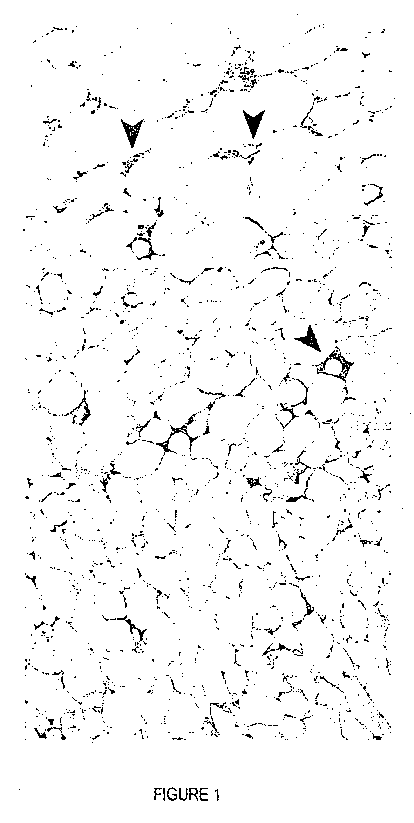

**Figure 1** shows a leptin induced blood vessel regression and apoptosis in adipose tissues of C57BL-*ob*^{-/-} mice. C57CB-*ob*^{-/-} mice were injected with murine leptin (2x1 µg/g) at time 0 and 9 h. Abdominal adipose tissues were removed at 24 and 48 h. Blood vessels were visualized in tissue sections by immunostaining with antibodies to Factor VIII (DAKO A/S, Denmark). Note that the number of stained blood vessels has decreased in 24 and 48 h post injection.

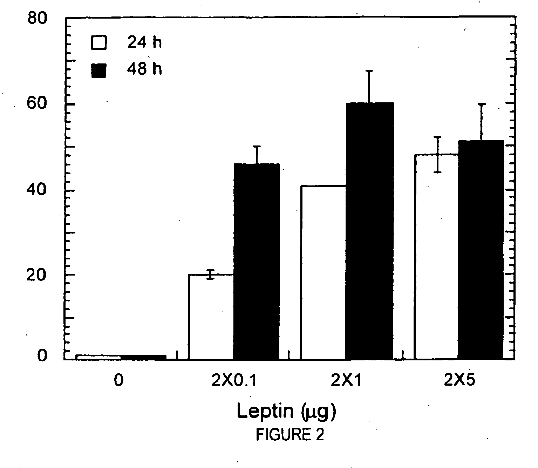

**Figure 2** shows a dose-response curve of the blood vessel regression in adipose tissue of *ob*^{-/-} mice 24 and 48 h post leptin injection.

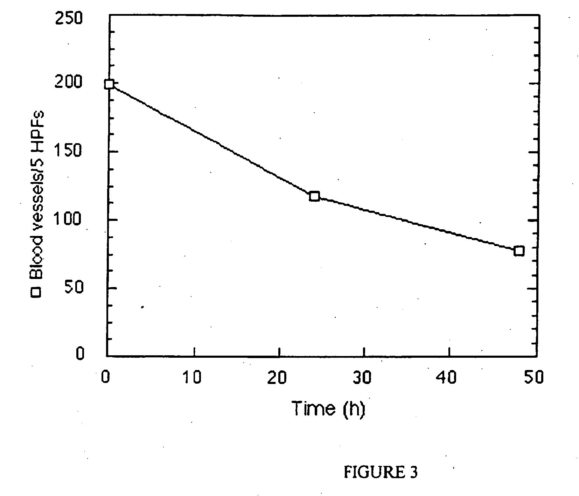

**Figure 3** shows a time course of the blood vessel regression in adipose tissues of *ob*^{-/-} mice injected with murine leptin (2x1 µg/g).

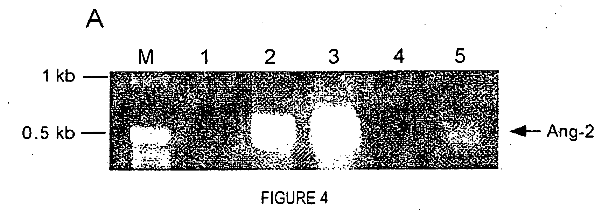

**Figure 4** shows a leptin-mediated induction of angiopoietin-2 (Ang2) as analyzed by reverse transcription-PCR of RNA from adipose tissues. Lane 1, control (no RNA); lane 2, RNA of adipose tissue from normal C57BL mouse; lane 3, RNA of adipose tissue from C57BL mouse injected with leptin (2x5 µg/g) lane 4, RNA of adipose tissue from C57BL-*ob*^{-/-} mouse; lane 5, RNA of adipose tissue from C57BL-*ob*^{-/-} mouse injected with leptin (2x5 µg/g). PCR reactions were terminated before saturation. PCR primers of Ang2, GeneBank Accession no. AF004326, corresponded to positions 637-657 (sense) and 1167-1147 (reverse).

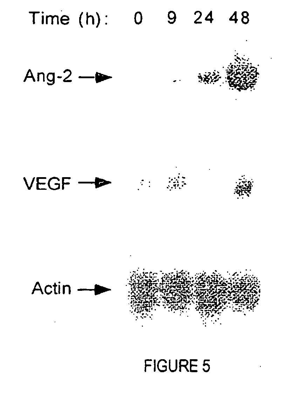

**Figure 5** shows a time course of Ang2 induction by leptin in adipose tissue of *ob*^{-/-} mice. Leptin (2x5 µg/g) was administered to C57BL-*ob*^{-/-} mice, total adipose RNA was extracted at the indicated times and analyzed by RNA blotting with probes to mouse Ang2, VEGF and actin.

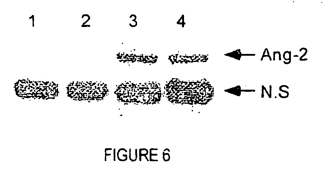

**Figure 6** shows a dose response of Ang2 induction in adipose tissue of *ob*^{-/-} mice. Leptin was administered to C57BL-*ob*^{-/-} mice and adipose proteins were extracted at 48 h. Ang2 was determined by immunoblot analysis (50 µg protein/lane) with specific antiserum (Santa Cruz Biotechnology, Santa Cruz, CA). The non-specific band (N.S.) serves for normalization of the immunoblot.

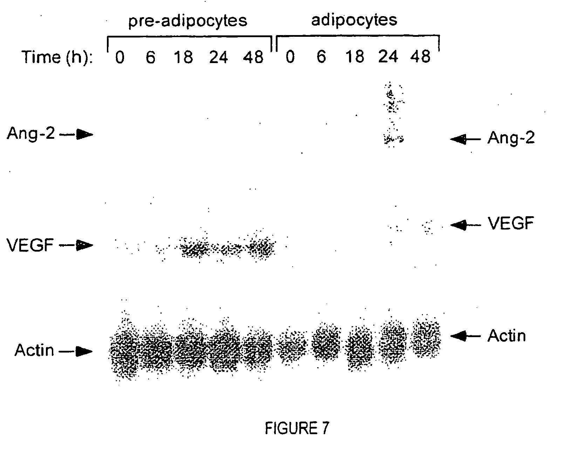

**Figure 7** shows induction of Ang2 by leptin in cultured adipocytes. Cultures of undifferentiated mouse 3T3-F442A pre-adipocytes and differentiated adipocytes were induced with leptin (1 µg/ml). Total RNA was extracted at different time points and subjected to RNA blotting with probes to mouse Ang2, VEGF and actin. Notice the punctuate induction of Ang2 in adipocytes at 24 h and the reduction in VEGF level following differentiation of pre-adipocytes into mature adipocytes.

### Detailed Description

Recently, leptin was reported to act as an angiogenic factor. It induced human umbilical vein endothelial cell proliferation in vitro, enhanced the formation of capillary-like tubes in vitro and induced neovascularization in corneas of mice and in a chic chorioallantoic membrane (M. R. Sierra-Honigmann, et al., Science 281, 1683-1686, 1998; A. Bouloumie, H. C. Drexler, M. Lafontan, R. Busse, Circ Res 83, 1059-1066, 1998). Although these studies suggested that leptin may induce angiogenesis at its site of production, the role of leptin as an angiogenic factor in the adipose tissue and in tumors has not yet been studied.

It has been found in accordance with the present invention that leptin, optionally together with other agents, acts as inducer of blood vessel regression in tissues and in tumors. Leptin potently induces the expression of the angiostatic factor angiopoietin-2 (Ang2) in various tissues, including adipose tissues and tumors. Ang2 is angiostatic in the absence of VEGF. Thus leptin is effective for modulating angiogenesis, and inhibiting unwanted angiogenesis, especially angiogenesis-related to tumor growth, adipose tissue growth and the estrous cycle.

The present invention includes the use of leptin, or homologues of leptin, or derivatives of leptin, optionally together with one or more inhibitors of VEGF production or VEGF action (hereinafter: "VEGF inhibitors") as inhibitors of tumor angiogenesis and modulators of angiogenesis in the female reproductive organs.

The present invention also includes the use of leptin, or homologues of leptin, or derivatives of leptin, together with VEGF inhibitors to induce adipose-tissue regression and to modulate angiogenesis in the female reproductive organs.

Administration of leptin, or homologues of leptin, or leptin derivatives, either alone or together with VEGF inhibitors to a human or animal with prevascularized metastasized tumors will prevent the growth or expansion of those tumors.

Administration of leptin, or homologues of leptin, or leptin derivatives, in combination with VEGF inhibitors or other inhibitors of angiogenesis to females will modulate angiogenesis in their reproductive organs.

Diseases and processes that are mediated by angiogenesis include, but are not limited to, hemangioma, solid tumors, blood borne tumors, leukemia, metastasis, telangiectasia, psoriasis, scleroderma, pyogenic granuloma, myocardial angiogenesis, Crohn's disease, plaque neovascularization, coronary collaterals, cerebral collaterals, arteriovenous malformations, ischemic limb angiogenesis, corneal diseases, rubeosis, neovascular glaucoma, diabetic retinopathy, retrolental fibroplasia, arthritis, diabetic neovascularization, macular degeneration, wound healing, peptic ulcer, Helicobacter related diseases, fractures, keloids, vasculogenesis, hematopoiesis, ovulation, menstruation, placentation, and cat scratch fever.

Administration of leptin, or homologues of leptin, or leptin derivatives, together with VEGF inhibitors or other inhibitors of angiogenesis to human or animal will reduce abberant angiogenesis associated with the aforesaid diseases more effectively than the VEGF inhibitor alone or other inhibitors of angiogenesis when applied without leptin.
It is also possible to modulate angiogenic processes by gene therapy as will be described hereinafter.

The present invention includes a use of leptin, or homologues of leptin, or a leptin derivatives, optionally together with VEGF inhibitors or other inhibitors of angiogenesis for the preparation of a medicament for treating an angiogenesis-mediated disease. The effective amount of leptin, or homologues of leptin, or a leptin derivatives, optionally together with VEGF inhibitors or other inhibitors of angiogenesis is administered to patients in a pharmaceutically acceptable composition.

It is to be understood that the present invention is contemplated to include the use of any homologues of leptin that induce endothelial inhibitory activity. Homologues of leptin refer to proteins, in which one or more of the amino acid residues of a natural leptin are replaced by different amino acid residues, or are deleted, or one or more amino acid residues are added to the natural sequence of leptin, without changing considerably the activity of the resulting products as compared with the wild type leptin. These homologues are prepared by known synthesis and/or by site-directed mutagenesis techniques, or any other known technique suitable therefor.

Any such homologue preferably has a sequence of amino acids sufficiently duplicative of that of leptin, such as to have substantially similar activity to leptin. One such activity is the ability of a leptin homologue to reduce the body weight of ob/ob mice. Thus, it can be determined whether any given homologue has substantially the same activity as leptin by means of routine experimentation.

In a preferred embodiment, any such mutein has at least 40% sequence identity or homology with the sequence of either leptin. More preferably, it has at least 50%, at least 60%, at least 70%, at least 80% or, most preferably, at least 90% sequence identity or homology thereto.

Homologues of leptin polypeptides, which can be used in accordance with the present invention, or nucleic acid coding therefor, include a finite set of substantially corresponding sequences as substitution peptides or polynucleotides which can be routinely obtained by one of ordinary skill in the art, without undue experimentation, based on the teachings and guidance presented herein. For a detailed description of protein chemistry and structure, see Schulz, G.E. et al., Principles of Protein Structure, Springer-Verlag, New York, 1978; and Creighton, T.E., Proteins: Structure and Molecular Properties, W.H. Freeman & Co., San Francisco, 1983, which are hereby incorporated by reference. For a presentation of nucleotide sequence substitutions, such as codon preferences, see Ausubel et al, *supra,* at §§ A.1.1-A.1.24, and Sambrook et al, *supra,* at Appendices C and D.

Preferred changes for homologues in accordance with the present invention are what are known as "conservative" substitutions. Conservative amino acid substitutions of leptin polypeptides may include synonymous amino acids within a group which have sufficiently similar physicochemical properties that substitution between members of the group will preserve the biological function of the molecule, Grantham, Science, Vol. 185, pp. 862-864 (1974). It is clear that insertions and deletions of amino acids may also be made in the above-defined sequences without altering their function, particularly if the insertions or deletions only involve a few amino acids, *e.g.,* under thirty, and preferably under ten, and do not remove or displace amino acids which are critical to a functional conformation, *e.g.,* cysteine residues, Anfinsen, "Principles That Govern The Folding of Protein Chains", Science, Vol. 181, pp. 223-230 (1973). Proteins and muteins produced by such deletions and/or insertions come within the purview of the present invention.

Preferably, the synonymous amino acid groups are those defined in Table I. More preferably, the synonymous amino acid groups are those defined in Table II; and most preferably the synonymous amino acid groups are those defined in Table III.

**TABLE I**

| **Preferred Groups of Synonymous** | |
|---|---|
| **Amino Acids** | **Amino Acid Synonymous Group** |
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gin | Glu, Lys, Asn, His, Thr, Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

**TABLE II**

| **More Preferred Groups of Synonymous** | |
|---|---|
| **Amino Acids** | **Amino Acid Synonymous Group** |
| Ser | Ser |
| Arg | His, Lys, Arg |
| Leu | Leu, Ile, Phe, Met |
| Pro | Ala, Pro |
| Thr | Thr |
| Ala | Pro, Ala |
| Val | Val, Met, Ile |
| Gly | Gly |
| Ile | Ile, Met, Phe, Val, Leu |
| Phe | Met, Tyr, Ile, Leu, Phe |
| Tyr | Phe, Tyr |
| Cys | Cys, Ser |
| His | His, Gln, Arg |
| Gln | Glu, Gln, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

**TABLE III**

| **Most Preferred Groups of Synonymous** | |
|---|---|
| **Amino Acids** | **Amino Acid Synonymous Group** |
| Ser | Ser |
| Arg | Arg |
| Leu | Leu, Ile, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, Ile, Leu |
| Trp | Met |

Examples of production of amino acid substitutions in proteins which can be used for obtaining homologues of leptin polypeptides or proteins for use in the present invention include any known method steps, such as presented in US patents RE 33,653, 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Shaw et al).

In another preferred embodiment of the present invention, any homologue of leptin has an amino acid sequence essentially corresponding to that of leptin. The term "essentially corresponding to" is intended to comprehend proteins with minor changes to the sequence of the natural protein which do not affect the basic characteristics of the natural proteins, particularly insofar as their ability to induce angiostatic activity. The type of changes which are generally considered to fall within the "essentially corresponding to" language are those which would result from conventional mutagenesis techniques of the DNA encoding these proteins, resulting in a few minor modifications, and screening for the desired activity in the manner discussed above.

It is to be understood that the present invention is contemplated to include the use of any derivatives of leptin that induce endothelial inhibitory activity when applied optionally together with a VEGF inhibitor or other inhibitors of angiogenesis. The present invention includes the use of an entire leptin protein, the use of derivatives of the leptin protein and the use of biologically active fragments of the leptin protein. Derivatives of leptin according to the invention have one or more chemical moieties attached thereto, including water-soluble polymers such as polyethylene glycol. Polyethylene glycol derivatized derivatives can be mono-, di-, tri- or tetrapegylated e. g., N-terminal monopegylated. Preferred N-terminal monopeglyated derivatives of leptin, optionally having a (pegylated) methionine at the N-terminus.

Various inhibitors of VEGF activity or VEGF production have been described and may be used in combination with leptin in order to inhibit angiogenesis. Among these inhibitors are 3,7-dimethyl-1-propargylxanthine(DMPX), an A2-antagonist, 7-(beta-hydroxyethyl)theophylline, 8-phenyltheophylline, the adenosine A2 receptor antagonist CSC ( 8-(3-chlorostyryl)caffeine), theobromine, an antagonistic VEGF variant, the soluble VEGF receptor sFLT-1, Tranilast, 8-(3-oxo-4,5,6-trihydroxy-3h-xanthen-9-yl)-1-naphthoic acid, suramin and platelet factor-4 (E. Hashimoto, et al., Biochem Biophys Res Commun 204, 318-24, 1994; S. Fischer, R. Knoll, D. Renz, G. F. Karliczek, W. Schaper, Endothelium 5, 155-165, 1997; H. Takagi, G. L. King, G. S. Robinson, N. Ferrara, L. P. Aiello, Invest Ophthalmol Vis Sci 37, 2165-2176, 1996; E. Barcz, et al., Oncol Rep 5, 517-520, 1998; G. Siemeister, et al., Proc Natl Acad Sci U S A 95, 4625-4629, 1998; W. Roeckl, et al., Exp Cell Res 241, 161-170, 1998, S. Komaya et al., Br J Pharmacol 127, 537-545, 1999: K. Igarashi et al., Int J Mol Med 2, 211-215, 1998; J. Waltenberger et al., J Mol Cell Cardiol 28, 1523-1529, 1996; S. Grengrinovitch et al., J Biol Chem 270, 15059-15065, 1995).

Various inhibitors of angiogenesis have been described and may be used in combination with leptin in order to inhibit angiogenesis more effectively than when used alone. Among these inhibitors are K1-5 (Cao, R. et al, Proc Natl Acad Sci U S A, 96, 5728-5733, 1999), angiostatin, endostatin, BB-94 and AGM-1470 (Bergers G. et al, Science284, 808-812, 1999).

Also comprised by the present invention is the use of expression vectors encoding leptin or leptin homologues, provided by gene therapy, together with inhibitors of VEGF action or production or other inhibitors of angiogenesis for preparing medicaments for inhibition of angiogenesis in tumors. Such medicaments can be employed in therapeutic methods involving intravenous, intraarterial, intraperitoneal, intramuscular, subcutaneous, nasal, oral or pulmonary delivery systems.

Also comprised by the present invention is the use of expression vectors encoding leptin or leptin homologues, provided by gene therapy, in combination with inhibitors of VEGF action or production or other inhibitors of angiogenesis for preparing medicaments for regression of adipose tissues. Such use may be useful in treating a disorder selected from the group consisting of diabetes, high blood pressure and high cholesterol and as part of combinative therapy with a medicament for treating such disorders. Such medicaments can be employed in therapeutic methods involving intravenous, intraarterial, intraperitoneal, intramuscular, subcutaneous, nasal, oral or pulmonary delivery systems.

The angiogenesis mediated diseases include, but are not limited to, obesity; solid tumors; blood born tumors such as leukemias; tumor metastasis; benign tumors, for example hemangiomas, acoustic neuromas, neurofibromas, trachomas, and pyogenic granulomas; rheumatoid arthritis; psoriasis; ocular angiogenic diseases, for example, diabetic retinopathy, retinopathy of prematurity, macular degeneration, corneal graft rejection, neovascular glaucoma, retrolental fibroplasia, rubeosis; Osler-Webber Syndrome; myocardial angiogenesis; plaque neovascularization; telangiectasia; hemophiliac joints, angiofibroma; and wound granulation.

Leptin, or homologues of leptin, or leptin derivatives, together with VEGF or other inhibitors of angiogenesis are useful in the preparation of medicaments for treatment of disease of excessive or abnormal stimulation of endothelial cells. These diseases include, but are not limited to, intestinal adhesions, Crohn's disease, arteriosclerosis, scleroderma, and hypertrophic scars, i.e., keloids.

Leptin, or homologues of leptin, or leptin derivatives, together with VEGF inhibitors or other inhibitors of angiogenesis may be used in combination with other compositions and procedures for preparing medicaments for the treatment of diseases. For example, a tumor may be treated conventionally with surgery, radiation or chemotherapy combined with leptin, or homologues of leptin, or leptin derivatives, optionally together with VEGF inhibitors or other inhibitors of angiogenesis and then leptin, or homologues of leptin, or leptin derivatives, optionally together with VEGF inhibitors or other inhibitors of angiogenesis may be subsequently administered to the patient to extend the dormancy of micrometastases and to stabilize and inhibit the growth of any residual primary tumor.

Additionally, Leptin, or homologues of leptin, or leptin derivatives, together with VEGF inhibitors or other inhibitors of angiogenesis, are combined with pharmaceutically acceptable excipients. Compositions suitable for parenteral administration include aqueous and non-aqueous sterile injection solutions which may contain anti-oxidants, buffers, bacteriostats and solutes which render the composition isotonic with the blood of the intended recipient; and aqueous and non-aqueous sterile suspensions which may include suspending agents and thickening agents. The compositions may be presented in unit-dose or multi-dose containers, for example, sealed ampoules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the kind previously described.

Compositions may optionally include sustained-release matrix, such as biodegradable polymers, to form therapeutic compositions. A sustained-release matrix, as used herein, is a matrix made of materials, usually polymers, which are degradable by enzymatic or acid/base hydrolysis or by dissolution. Once inserted into the body, the matrix is acted upon by enzymes and body fluids. The sustained-release matrix desirably is chosen from biocompatible materials such as liposomes, polylactides (polylactic acid), polyglycolide (polymer of glycolic acid), polylactide co-glycolide (co-polymers of lactic acid and glycolic acid) polyanhydrides, poly(ortho)esters, polypeptides, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides and polyvinylpyrrolidone.

A preferred biodegradable matrix is a matrix of one of either polylactide, polyglycolide, or polylactide co-glycolide (co-polymers of lactic acid and glycolic acid). The polymers being implanted in the vicinity of where drug delivery is desired, for example, at the adipose tissue or at a site of a tumor or implanted, so that the leptin, or leptin derivatives, optionally together with VEGF inhibitors or other inhibitors of angiogenesis is slowly released systemically. The biodegradable polymers and their use are described, for example, in detail in Brem et al., J. Neurosurg. 74:441-446 (1991), which is hereby incorporated by reference in its entirety.

The angiogenesis-modulating pharmaceutical compositions according to the present invention may be a solid, liquid or aerosol and may be administered by any known route of administration. Examples of solid therapeutic compositions include pills, creams, and implantable dosage units. The pills may be administered orally; the therapeutic creams may be administered topically. The implantable dosage units may be administered locally, for example at a tumor site, or may be implanted for systemic release of the therapeutic angiogenesis-modulating composition, for example subcutaneously. Examples of liquid compositions include compositions adapted for injection subcutaneously, intravenously, intraarterially, and compositions for topical and intraocular administration. Examples of aerosol compositions include inhaler composition for administration to the lungs.

It should be understood that in addition to the ingredients, specifically mentioned above, the compositions according to the present invention may include other agents conventional in the art having regard to the type of composition in question. Optionally, cytotoxic agents may be incorporated or otherwise combined with leptin, or homologues of leptin, or leptin derivatives, optionally together with VEGF inhibitors or other inhibitors of angiogenesis, to provide dual therapy to the patient.

The compositions according to the invention can be administered by standard routes. In general, the combinations may be administered by the topical (including buccal and sublingual), or parenteral (including subcutaneous, intraperitoneal, intramuscular, intravenous, intradermal, intracerebral, intracerebroventricular, intracranial, intraspinal, intratracheal, and epidural), transdermal, intravaginal, intrauterine, oral, rectal, ophthalmic (including intravitreal or intracameral), or intranasal, administration.

Osmotic minipumps may also be used to provide controlled delivery of high concentrations of leptin, or leptin derivatives, optionally together with VEGF inhibitors or other inhibitors of angiogenesis through cannulae to the site of interest, such as directly into a metastatic growth or into the vascular supply to that tumor.

The dosage of the leptin, or leptin derivatives, together with VEGF inhibitors or other inhibitors of angiogenesis of the present invention will depend on the disease state or condition being treated and other clinical factors such as weight and condition of the human or animal and the route of administration of the compound. For treating humans or animals, between approximately 0.5 mg/kilogram to 10 mg/kilogram of the leptin or leptin homologue or leptin derivative can be administered, optionally together with a suitable dose of a VEGF inhibitor or inhibitor of VEGF production or other inhibitors of angiogenesis. Depending upon the half-life of the leptin or leptin homologue or leptin derivative in the particular animal or human, the leptin or leptin homologue or leptin derivative can be administered between several times per day to once a week. Preferred unit dosage compositions are those containing a daily dose or unit, daily sub-dose, or an appropriate fraction thereof, of the administered ingredient. The methods of the present invention contemplate single as well as multiple administrations, given either simultaneously or over an extended period of time.

The leptin or leptin homologue or leptin derivative compositions may conveniently be presented in unit dosage form and may be prepared by conventional pharmaceutical techniques. Such techniques include the step of bringing into association the active ingredient and the pharmaceutical carrier(s) or excipient(s). In general, the compositions are prepared by uniformly and intimately bringing into association the active ingredient with liquid carriers or finely divided solid carriers or both, and then, if necessary, shaping the product.

Preferred unit dosage compositions are those containing a daily dose or unit, daily sub-dose, or an appropriate fraction thereof, of the administered ingredient. It should be understood that in addition to the ingredients, specifically mentioned above, the compositions of the present invention may include other agents conventional in the art having regard to the type of composition in question.

It is to be understood that the present invention has application for both human and veterinary use.

This invention is further illustrated by the following examples.

### Examples

### Example 1

### Induction of blood vessel regression by leptin

To test leptin's effect on blood vessel homeostasis in adult adipose tissue, murine leptin (0.1-5 µg/g) was injected ip at time 0 and 9 h to 8-10 weeks old obese (C57BL-ob^{-/-}) female mice, lacking endogenous leptin. A noticeable weight loss was observed at 48 h in mice receiving ≥ 2x1 µg/g leptin (65.4±0.5 g vs. 62.7±1.0 g, n=6). Abdominal fat was removed and fixed 24 and 48 h after the first injection, and blood vessels were counted after staining paraffin sections with antibodies to Factor VIII (D.D. Wagner et al. J Cell Biol 95, 355-360 (1982)). A significant reduction in the number of blood vessels was observed (198±1 vessels per 5 high power fields (HPFs, x400) in control mice; 159±2.5 vessels per 5 HPFs in leptin-treated mice (2x1 µg/g) at 24 h and 106±7.5 vessels per 5 HPFs at 48 h. **Figure 1** shows micrographs of blood vessels in adipose tissue sections. **Figure 2** shows the dose-response curve of the blood vessel regression and **Figure 3** shows the time course of this regression.

### Example 2

### Leptin induces Angiopoietin 2 (Ang2) in adipose tissues

The mechanism by which leptin induced the blood vessel regression in adipose tissues was studied by measuring its effect on the expression level of angiogenic and angiostatic factors. Total RNA was isolated from adipose tissue of C57BL and C57BL-*ob*^{-/-} mice at time 0 and 24 h after the first leptin administration. Total RNA was isolated with the TRI reagent. Reverse transcription was carried out in 20 µl volume using RNase H⁻ reverse transcriptase (SuperScript II, GIBCO-BRL) with 1 µg (N)₆ random primer (New England Biolabs) according to the manufacturer's instructions. Aliquot (2 µl) of the reverse transcription product was used for PCR with VENT DNA polymerase (New England Biolabs) and the following sense and antisense primers: muAng-2 mRNA, GeneBank Accession No. AF4326 nucleotides 637-657 and 1147-1167; muVEGF, GeneBank Accession No. M95200 nucleotides 385-406 and 962-980; muActin mRNA, GeneBank Accession No. J00691 nucleotides 1670-1691 and 2452-2431. PCR reactions were terminated before saturation. It was found that Ang2 mRNA is expressed in adipose tissue of normal mice and not in that of the *ob*^{-/-} mice. Furthermore, injection of leptin induced the expression of Ang2 in both types of mice (**Figure 4**). These results demonstrate that leptin is a potent inducer of the angiostatic factor Ang2.

The levels and induction of Ang2 mRNA by leptin in the adipose tissue of *ob*^{-/-} mice was then studied by RNA blotting with specific probes to Ang2 and VEGF. Total RNA from adipose tissue was isolated with the TRI reagent kit (Molecular Research Center Inc.). Samples of RNA (15 µg) were resolved by electrophoresis through 1% agarose gel in MOPS-formaldehyde buffer, transferred to nylon membrane (Hybond N, Amersham) in 20XSSC buffer and the membrane was then heated for 2 hours at 80°C in a vacuum oven. The membrane was pre-hybridized (6 h, 42°C) with denatured Salmon-sperm DNA (100 µg/ml in 50% formamide, 5xSSC, 4xDenhard's solution and 0.5% SDS). A [³²P]dCTP DNA probe (1x10⁶ cpm/ml), prepared by random priming, was then added and hybridization continued for 18 hours at 42°C. The membrane was then washed at room temperature (1xSSC, 0.1% SDS twice, 0.25xSSC, 0.1% SDS and 0.1xSSC, 0.1% SDS twice, 30 min. each wash) and autoradiographed. Blots were then re-hybridized with ³²P-labeled probe corresponding to mouse actin to show equal amounts of RNA in the blot. A significant induction of Ang2 was obtained following leptin administration (2.9±0.4 fold, P<0.05, n=3 at 24 h and 16.0±0.31 fold, P<0.01, n=3 at 48 h; **Figure 5**). The kinetics of Ang2 expression corresponded to that of the apoptosis and blood vessel regression. The level of VEGF mRNA was only slightly induced (1.4±0.1 fold, n=3 at 48 h; **Figure 5**).

The dose-response of leptin-induced Ang2 in adipose tissues was studied by immunoblotting 48 h after the first injection of leptin. Cell extracts from adipose tissue were isolated using the TRI reagent kit (Molecular Research Center Inc.) in parallel to total RNA extraction. Fifty micrograms protein were separated on 10% SDS-polyacrylamide gel. Immunoblot analysis was carried out with 5 µg of a specific goat anti-human Ang-2 antibody. Ang2 was below the level of detection in adipose tissue of control *ob*^{-/-} mice, whereas administration of 2x1 µg/g leptin was sufficient for high level induction of Ang2 **(****Figure 6****)**.

### Example 3

### Leptin induces Angiopoietin 2 (Ang2) in cultured adipocytes

Several peripheral activities of leptin were previously reported (M. R. Sierra-Honigmann, et al., Science 281, 1683-1686, 1998; A. Bouloumie, H. C. Drexler, M. Lafontan. R. Busse, Circ Res 83, 1059-1066,1998; B. Cohen, D. Novick, M. Rubinstein, Science 274, 1185-1188, 1996; D. Barkan, et al., Endocrinology 140, 1731-1738, 1999). To test if leptin may act directly on adipocytes, we studied the effect of leptin on murine 3T3-F442A pre-adipocytes, known to give rise to adipose-like tissue upon implantation in athymic mice (H. Green, O. Kehinde, J Cell Physiol 101, 169-171, 1979); S. Mandrup, T. M. Loftus, O. A. MacDougald, F. P. Kuhajda, M. D. Lane, Proc Natl Acad Sci US A 94, 4300-4305,1997). Swiss 3T3 F442A murine pre-adiopcytes (H. Green, O. Kehinde. Cell 5, 19-27, 1975) were grown in DMEM (GIBCO) and 10% calf serum. For differentiation, confluent cells were maintained in DMEM supplemented with 10% fetal bovine serum (FBS) for six days. Medium was replaced every 48 hours. By the end of the period most of the cells acquired the characteristic adipocyte morphology as determined by biochemical and morphological criteria. Leptin (1 µg/ml) was added to cultures of differentiated and non-differentiated cells. RNA was isolated from the cultured cells as described for the adipose tissues of Example 2 and subjected to RNA blot analysis. It was found that leptin induced Ang2 mRNA expression in differentiated 3T3-F442A adipocytes and not in pre-adipocytes. Ang2 mRNA appeared punctuate at 24 h. VEGF mRNA was constitutively expressed in pre-adipocytes and was further induced by leptin. The level of VEGF mRNA was significantly lower in mature adipocytes and was not significantly induced by leptin (**Figure 7**). These result suggest that leptin induces an angiostatic signal in mature adipocytes and angiogenic signals in pre-adipocytes.

### Example 4

### Effect of leptin plus a VEGF inhibitor on adipose mass reduction

The angiostatic activity of leptin-induced Ang2 is reversed in the presence of VEGF. Furthermore, a modest induction of VEGF by leptin was noticed in the previous examples. Therefore, murine leptin (0.1-5 µg/g) is injected ip at time 0 and 9 h to 8-10 weeks old obese (C57BL-ob^{-/-}) female mice, lacking endogenous leptin. In parallel, 8-10 weeks old obese (C57BL-ob^{-/-}) female mice were injected at times 0 and 9 h ip with murine leptin (0.1-5 µg/g) together with the adenosine 2 receptor antagonist CSC, known to function as a VEGF inhibitor (H. Takagi, G. L. King, G. S. Robinson, N. Ferrara, L. P. Aiello, *Invest Ophthalmol Vis Sci* **37**, 2165-2176, 1996). A noticeable weight loss was observed at 48 h in mice receiving ≥ 2x1 µg/g leptin alone (65.4±0.5 g vs. 62.7±1.0 g, n=6). A significantly higher weight loss is observed in mice treated with a combination of leptin and CSC.

## Claims

1. The use of leptin or a leptin homologue or derivative optionally together with an inhibitor of VEGF action or of VEGF synthesis and/or an inhibitor of angiogenesis, in the preparation of a medicament for inhibiting angiogenesis by inducing Ang-2, wherein said leptin homologue has similar activity to leptin, and wherein said leptin derivative induces endothelial inhibitory activity.

2. The use according to claim 1 for inhibiting angiogenesis in adipose tissue.

3. The use according to claim 1 or 2, including an angiogenesis inhibitor.

4. The use according to anyone of the preceding claims wherein the VEGF inhibitor is selected from DMPX, an A2-antagonist 7-(betahydroxyethyl)theophylline, 8-phenyltheophylline, the adenosine A2 receptor antagonist CSC, theobromine, an antagonistic VEGF variant, sFLT-1, Tranilast,8-(3-oxo-4,5,6-trihydroxy-3h-xanthen-9-yl)-1-naphthoic acid, suramin and platelet factor-4.

5. A pharmaceutical composition comprising leptin or a leptin homologue or derivative together with an inhibitor of VEGF action or of VEGF synthesis and/or an inhibitor of angiogenesis, wherein said leptin homologue has similar activity to leptin, and wherein said leptin derivative induces endothelial inhibitory activity.

6. A pharmaceutical composition according to claim 5 for modulating angiogenic processes.

7. A pharmaceutical composition according to claim 6 for inhibiting angiogenesis.

8. A mixture comprising leptin and a VEGF inhibitor.

## Patentansprüche

1. Verwendung von Leptin oder einem Leptinhomolog oder -derivat, gegebenenfalls zusammen mit einem Hemmer der VEGF-Wirkung oder der VEGF-Synthese und/oder einem Hemmer der Angiogenese, bei der Herstellung eines Medikamentes zum Hemmen der Angiogenese durch Induzieren von Ang-2, wobei das Leptinhomolog eine ähnliche Wirkung wie Leptin aufweist und wobei das Leptinderivat eine endotheliale Hemmwirkung induziert.

2. Verwendung nach Anspruch 1 zum Hemmen der Angiogenese in Fettgewebe.

3. Verwendung nach Anspruch 1 oder 2, die einen Angiogenesehemmer einschließt.

4. Verwendung nach einem der vorangehenden Ansprüche, wobei der VEGF-Hemmer ausgewählt ist aus DMPX, einem A2-Antagonisten, 7-(Betahydroxyethyl)theophyllin, 8-Phenyltheophyllin, dem Adenosin A2-Rezeptorantagonisten CSC, Theobromin, einer antagonistischen VEGF-Variante, sFLT-1, Tranilast, 8-(3-Oxo-4,5,6-trihydroxy-3h-xanthen-9-yl)-1-naphthoesäure, Suramin und Plättchenfaktor-4.

5. Pharmazeutische Zusammensetzung umfassend Leptin oder ein Leptinhomolog oder -derivat zusammen mit einem Hemmer der VEGF-Wirkung oder der VEGF-Synthese und/oder einem Hemmer der Angiogenese, wobei das Leptinhomolog eine ähnliche Wirkung wie Leptin aufweist und wobei das Leptinderivat eine endotheliale Hemmwirkung induziert.

6. Pharmazeutische Zusammensetzung nach Anspruch 5 zum Modulieren von angiogenen Prozessen.

7. Pharmazeutische Zusammensetzung nach Anspruch 6 zum Hemmen der Angiogenese.

8. Mischung, umfassend Leptin und einen VEGF-Inhibitor.

## Revendications

1. Utilisation d'une leptine, ou d'un homologue ou d'un dérivé de leptine, en présence, optionnellement, d'un inhibiteur de l'action du facteur VEGF ou de la synthèse du facteur VEGF et/ou à un inhibiteur de l'angiogenèse, dans la préparation d'un médicament conçu pour inhiber l'angiogenèse par induction de Ang-2, ledit homologue de leptine possédant une activité similaire à celle d'une leptine et ledit dérivé de leptine induisant une activité inhibitrice endothéliale.

2. Utilisation conforme à la revendication 1, pour inhiber l'angiogenèse dans un tissu adipeux.

3. Utilisation conforme à la revendication 1 ou 2, incluant un inhibiteur d'angiogenèse.

4. Utilisation conforme à l'une des revendications précédentes, pour laquelle l'inhibiteur de VEGF est choisi parmi les suivants : le DMPX, l'antagoniste de récepteurs A2 7-(β-hydroxy-éthyl)-théophylline, la 8-phényl-théophylline, l'antagoniste de récepteurs A2 de l'adénosine CSC, la théobromine, un variant de VEGF à caractère antagoniste, le récepteur sFLT-1, le tranilast, l'acide 8-(3-oxo-4,5,6-trihydroxy-3H-xanthén-9-yl)-napht-1-oïque, le suramin, et le facteur plaquettaire 4.

5. Composition pharmaceutique comprenant une leptine, ou un homologue ou un dérivé de leptine, en présence d'un inhibiteur de l'action du facteur VEGF ou de la synthèse du facteur VEGF et/ou d'un inhibiteur de l'angiogenèse, ledit homologue de leptine possédant une activité similaire à celle d'une leptine et ledit dérivé de leptine induisant une activité inhibitrice endothéliale.

6. Composition pharmaceutique conforme à la revendication 5, conçue pour moduler les processus angiogéniques.

7. Composition pharmaceutique conforme à la revendication 6, conçue pour inhiber l'angiogenèse.

8. Mélange comprenant une leptine et un inhibiteur de VEGF.
